# EUROPEAN PATENT APPLICATION

(11) **EP 2 529 736 A1**
(43) Date of publication of application: **05.12.2012**
(21) Application number: 11737179.9
(22) Date of filing: 28.01.2011
(51) Int. Cl.: A61K 31/352, A61K 36/00, A61P 3/10, A61P 17/16, A61P 17/18, A61P 29/00, A61P 37/08, A61P 39/06

(54) **EXPRESSION PROMOTER FOR REDOX-RELATED FACTOR**

(30) Priority: 29.01.2010 US 299589 P
(71) Applicant: Shiseido Co., Ltd., Tokyo 104-8010 (JP); The General Hospital Corporation, Boston, Massachusetts 02114 (US)
(72) Inventor: SUGAHARA, Misato, Yokohama-shi Kanagawa 224-8558 (JP); KATSUTA, Yuji, Yokohama-shi Kanagawa 224-8558 (JP); NAKANISHI, Jotaro, Yokohama-shi Kanagawa 224-8558 (JP); LEE, Sam W., Newton, Massachusetts 02459 (US)
(74) Representative: Henkel, Breuer & Partner
(86) International application number: PCT/JP2011/051811
(87) International publication number: WO 2011/093469

(57) **Abstract**

The present invention provides an agent for enhancing expression of a redox-associated factor, which comprises kaempferol, quercetin or glycosides thereof, or any combination thereof as an active ingredient, and an agent for enhancing expression of a redox-associated factor, which comprises ginkgo leaf extract, Kaempferia galanga extract or any combination thereof as an active ingredient.

SEQUENCE LISTING

<110>  SHISEIDO CO. LTD; THE GENERAL HOSPITAL CORPORATION

<120>  AN AGENT FOR ENHANCING THE EXPRESSION OF REDOX-ASSOCIATED FACTORS

<130>  Y826-PCT

<150>  US 61/299,589
<151>  2010-01-29

<160>  12

<170>  PatentIn version 3.1

<210>  1
<211>  29
<212>  DNA
<213>  Artificial

<220>
<223>  forward primer

<400>  1
tgctggcaat aggaagagat ggcttgcac                                29

<210>  2
<211>  32
<212>  DNA
<213>  Artificial

<220>
<223>  reverse primer

<400>  2
gcaatcttcc tgcctgcctg gattgcaact gg                            32

<210>  3
<211>  30
<212>  DNA
<213>  Artificial

<220>
<223>  forward primer

<400>  3
tcggtcctta cagccgctcg tcagactcca                               30

<210>  4
<211>  30
<212>  DNA
<213>  Artificial

<220>
<223>  reverse primer

<400>  4
aggcccacac cacgtggctg agaagtcaac                                        30

<210>  5
<211>  30
<212>  DNA
<213>  Artificial

<220>
<223>  forward primer

<400>  5
gatcctgtca gccctgggtt ctaagacatc                                        30

<210>  6
<211>  28
<212>  DNA
<213>  Artificial

<220>
<223>  reverse primer

<400>  6
taaccatgct gacttccaag cccgacaa                                          28

<210>  7
<211>  28
<212>  DNA
<213>  Artificial

<220>
<223>  forward primer

<400>  7
atcacagcca ccaaccacac taacgaga                                          28

<210>  8
<211>  30
<212>  DNA
<213>  Artificial

<220>
<223>  reverse primer

<400>  8
gttactgcag agctccaatc tgctttagcc                                    30

<210>  9
<211>  30
<212>  DNA
<213>  Artificial

<220>
<223>  forward primer

<400>  9
acagaggaaa ctctgcattc tcgcttcctg                                    30

<210>  10
<211>  29
<212>  DNA
<213>  Artificial

<220>
<223>  reverse primer

<400>  10
cacagacaag gccaggactc gtttgtacc                                     29

<210>  11
<211>  28
<212>  DNA
<213>  Artificial

<220>
<223>  forward primer

<400>  11
ggccatggac aagctgagga agatgctg                                      28

<210>  12
<211>  29
<212>  DNA
<213>  Artificial

<220>
<223>  reverse primer

<400> 12
tgcatcgtgc acataagcct cgttatccc 29

## Description

### Technical Field

This invention relates to an agent for enhancing expression of a redox-associated factor, which comprises kaempferol, quercetin, glycosides thereof or any combination thereof as an active ingredient, and to an agent for enhancing expression of a redox-associated factor, which comprises ginkgo leaf extract, Kaempferia galanga extract or any combination thereof as an active ingredient.

### Background Art

In organisms, redox regulations are carried out so as to maintain their existence and homeostasis. Such in vivo redox regulations are mainly based on reversible oxidation-reduction reactions on a thiol group of a cysteine residue in intracellular proteins, which may regulate various cellular functions. It has been known that a factor associated with the redox regulations (i.e., redox-associated factor) maintains the reduction condition of the environment inside a cell, and thus a redox-associated factor may play an essential role in cell viability and an important role in elimination of reactive oxygen species. If such a redox-associated factor does not function properly, endogenous reactive oxygen species are excessively produced, and thus cells can be exposed to excessive oxidative stress. Such excessive oxidative stress may oxidize biological macromolecules such as DNA, proteins and lipids, and damage them thereby causing various diseases and aging. Therefore, recently, redox-associated factors, which may have an effect of eliminating the reactive oxygen species causing oxidative stress, have been aggressively studied.

As a redox-associated factor which has an effect of preventing an organism from oxidative stress, a glutathione superfamily has been known. Glutathione is a tripeptide in mammals, and has been reported to have a reduction action resulting from intramolecular thiol groups thereof, coenzymatic function, function for generating mercapturic acid and other detoxification mechanisms, protective effect on thiol enzymes and other cellular components, function for facilitating the elimination of deleterious materials, anti-allergic effect resulting from enhanced cholinesterase activity, enzyme-activating action and the like. However, since glutathione forms a crystalline precipitate in a formulation, cells cannot directly absorb a glutathione molecule. Therefore, an ingredient for enhancing the intracellular production of glutathione can be quite useful. As such an ingredient, γ-tocopherol or δ-tocopherol has been known, and glutathione-production enhancing agents comprising such an ingredient has been disclosed (Japanese Unexamined Patent Publication (Kokai) No. 2007-284430 (Patent Publication 1)).

In addition, as another redox-associated factor, a thioredoxin superfamily, which also has an effect of preventing an organism from oxidative stress, has been known (Saibou Kougaku, vol.25. No.2, 2006, p.143-148 (Non-patent publication 1)). It has been reported that thioredoxin transgenic mice have a longer life-span (Mitsui A, et al.: Antioxid Redox Signal (2002) 4: 693-696 (Non-patent publication 2)), that thioredoxin provides resistance against cerebral infarction, diabetes or the like (Takagi Y, et al.: Proc Natl Acad Sci USA (1999) 96: 4131-4136 (Non-patent publication 3), Hotta M, et al.: J Exp Med (1998) 188: 1445-1451 (Non-patent publication 4)), and that thioredoxin is effective for allergic dermatitis (Japanese Unexamined Patent Publication (Kokai) No. 2007-269671 (Patent publication 2)). In addition, thioredoxin has been widely used as an active oxygen eliminating agent (Japanese Unexamined Patent Publication (Kokai) No. 9-157153 (Patent publication 3)). However, since the thioredoxin superfamily is a macromolecule protein, even if such a compound is directly administered to a subject, it cannot be absorbed by cells. Therefore, it is desirable to enhance the in vivo expression of thioredoxin superfamily. However, as an ingredient which can enhance the expression of the family, only an Artemisia extract or a green perilla extract has been known (International publication WO2006/033351 (Patent publication 4)).

As mentioned above, even if a redox-associated factor, i.e., a compound belonging to the glutathione superfamily or thioredoxin superfamily, is directly administered to a subject, it cannot be efficiently absorbed by cells, and thus a redox-regulative function or anti-oxidative function cannot be achieved in the subject. Although it has been known that the expression of a redox-associated factor is enhanced by stress, such as UV irradiation, few chemical substances acting safely and efficiently on an organism have been known, and the mechanism of in vivo expression of a redox-associated factor has not been fully elucidated. Therefore, it is still necessary to find a safe and efficient ingredient for enhancing in vivo expression of a redox-associated factor.

### Citation List

### LIST OF PATENT PUBLICATIONS

Patent Publication 1: JP 2007-284430
Patent Publication 2: JP 2007-269671
Patent Publication 3: JP 9-157153
Patent Publication 4: International publication No. 2006/033351

### LIST OF NON-PATENT PUBLICATIONS

Non-patent Publication 1: Saibou Kougaku, vol.25, No.2, 2006, p143-148
Non-patent Publication 2: Mitsui A, et al.: Antioxid Redox Signal (2002) 4: 693-696
Non-patent Publication 3: Takagi Y, et al.: Proc Natl Acad Sci USA (1999) 96: 4131-4136
Non-patent Publication 4: Hotta M, et al.: J Exp Med (1998) 188: 1445-1451

### Summary of Invention

### PROBLEMS TO BE SOLVED BY THE INVENTION

The problems to be solved by the present invention are to safely and efficiently reduce oxidative stress in an organism (subject), to maintain the subject in good health and to prevent various diseases and symptoms resulting from oxidative stress by enhancing in vivo expression of a redox-associated factor.

### MEANS FOR SOLVING THE PROBLEMS

The inventors have discovered that kaempferol and quercetin belonging to flavonols, can enhance in vivo expression and activity of a redox-associated factor, i.e., compounds belonging to the glutathione superfamily and thioredoxin superfamily. Unexpectedly, among flavonols, such a function is specific to kaempferol and quercetin, but myricetin which has a similar chemical structure does not have such a function. Compared with the case of directly applying a molecule of the glutathione superfamily or the thioredoxin superfamily, applying kaempferol or quercetin can effectively enhance the expression and activity of endogenous redox-associated factors, i.e., compounds belonging to the glutathione superfamily or thioredoxin superfamily. Therefore, various endogenous oxidative stresses can be alleviated by the present invention. Flavonols including kaempferol and quercetin are innoxious and safely consumable compounds for an organism, and are widely distributed in plants such as tea leaves or the like. Therefore, endogenous excessive reactive oxygen species can be eliminated without providing any stress to an organism by using kaempferol and quercetin as an active ingredient for enhancing in vivo expression of a redox-associated factor.

Therefore, the present application comprises the following embodiments:
[1] An agent for enhancing expression of a redox-associated factor, which comprises kaempferol, quercetin, glycosides thereof or any combination thereof as an active ingredient.
[2] The agent for enhancing expression of a redox-associated factor according to [1], wherein the redox-associated factor is one or more substances selected from the group consisting of thioredoxin, thioredoxin reductase, glutathione reductase, and glutaredoxin.
[3] An agent for enhancing expression of a redox-associated factor, which comprises ginkgo leaf extract, Kaempferia galanga extract, or any combination thereof as an active ingredient.
[4] The agent for enhancing expression of a redox-associated factor according to [3], wherein the redox-associated factor is one or more substances selected from the group consisting of thioredoxin, thioredoxin reductase, glutathione reductase, and glutaredoxin.
[5] Use of kaempferol, quercetin, glycosides thereof or any combination thereof in the preparation of an agent for enhancing expression of a redox-associated factor.
[6] Use of ginkgo leaf extract, Kaempferia galanga extract, or any combination thereof in the preparation of an agent for enhancing expression of a redox-associated factor.

### Technical Advantage of the Invention

Enhancing in vivo expression of a redox-associated factor can safely and efficiently reduce endogenous reactive oxygen species, and protect an organism from oxidative stress, whereby the organism can be maintained in good health, and various diseases and symptoms resulting from the oxidative stress can be avoided.

### Brief Description of Drawings

Fig.1 shows enhancement for in vivo expression of a redox-associated factor by kaempferol.
Fig.2 shows enhancement for the expression of a redox-associated factor in skin-equivalent models by kaempferol.
Fig.3 shows a comparison of an enhancing effect by flavonoid compounds on expression of thioredoxin superfamily.
Fig.4 shows enhancement for the expression of thioredoxin reductase by ginkgo leaf extract and Kaempferia galanga extract.
Fig.5 shows a enhancing effect for thioredoxin reductase activity by kaempferol and quercetin.
Fig.6 shows a suppressing effect for producing inflammatory cytokine (IL1β) by kaempferol, ginkgo leaf extract and Kaempferia galanga extract.

### Description of Embodiments

The first embodiment of the present invention provides an agent for enhancing expression of a redox-associated factor, which comprises kaempferol, quercetin, glycosides thereof or any combination thereof as an active ingredient.

The term "redox-associated factor" as used herein refers to a factor which oxidatively or reductively reacts with a thiol group of a cysteine residue of a protein in an organism such as a mammal, preferably a human being, and maintains the reduction condition of the environment inside the cell. In activating cells and processing signaling messengers, i.e., in signal transduction mechanism, modifications of a protein-protein interaction by oxidizing a thiol group of a cysteine residue in an intracellular protein play an important role. This oxidation/reduction (redox) of a thiol group of a cystein residue is associated with regulations of various life phenomena, such as transcription of genes, regulations of intracellular location, synthesis and decomposition of proteins, cell proliferation and cell death. As such an endogenous redox-associated family, glutathione superfamily and thioredoxin superfamily have been known, and each of them includes a series of compounds. The redox-associated factor has been known to be involved in the elimination of reactive oxygen species, which are produced and are present in vivo.

Thioredoxin superfamily includes, in particular, thioredoxin, thioredoxin reductase and the like. Thioredoxin (TRX) was found as a coenzyme providing hydrogen to ribonucleotide reductase which is an enzyme essential for DNA synthesis in E. coli. The TRX is a protein with approximately 12 kDa, which is conserved in a variety of organisms such as prokaryotes and human beings, has a steric structure consisting of four β-strands and three α-helices, and has the active site of - Cys-Gly-Pro-Cys-. The TRX has an oxidized form (S-S) which has a disulfide bond between two cysteine residues of the active site, and a reduced form (-SH, SH-) which cleaves a disulfide bond of a substrate protein and returns the oxidized form per se. The oxidized form of TRX is reduced by NADPH and thioredoxin reductase, and the reduced form is regenerated. As one of the substrate proteins, an H₂O₂ eliminating enzyme superfamily, which is generally referred to as peroxiredoxin, has been known. A reduced form of peroxiredoxin reduces H₂O₂. An oxidized peroxiredoxin functions as a substrate and is reduced by thioredoxin. An oxidized thioredoxin is reduced by thioredoxin reductase. Therefore, the thioredoxin superfamily plays an important role as a detoxifying mechanism for reactive oxygen species.

The glutathione superfamily includes, in particular, glutathione, glutathione reductase, glutaredoxin and the like. Glutathione is a tripeptide which consists of glutamic acid, cysteine, and glycine, and a large amount of Glutathione is found in cells as a nonprotein thiol ingredient. Further, it has been known as a major antioxidant in hydrophilic fraction of cells. Glutathione reacts with reactive oxygen species (e.g., superoxide or hydrogen peroxide) to be a stable glutathione radical and form a dimer (GSSG: an oxidized form of glutathione), which is further regenerated as GSH (a reduced form of glutathione) after glutathione reductase transfers electrons from NADPH to GSSG. Glutaredoxin has the active site of -Cys-Pro-Tyr-Cys-, which is common to thioredoxin, and thus belongs to the thioredoxin superfamily. Glutaredoxin also has an oxidized form (S-S) having a disulfide bond between the two cysteine residues of the active site and a reduced form (-SH, SH-). Similar to thioredoxin, the reduced form cleaves a disulfide bond of a substrate protein and returns the oxidized form per se. Although its biological activities are not completely understood, glutaredoxin is known to be associated with in vivo redox regulations and oxidative stress responses.

Therefore, excessive amounts of endogenous reactive oxygen species can be reduced by activating redox-associated factor(s), such as thioredoxin, thioredoxin reductase, glutathione reductase and glutaredoxin, enhancing the expression of them, and thus an organism can be protected from the oxidative stress.

As described above, the inventors recently and surprisingly discovered that kaempferol and quercetin can activate the above redox-associated factors, and enhance their expression. Flavonols including kaempferol and quercetin have the following formula. 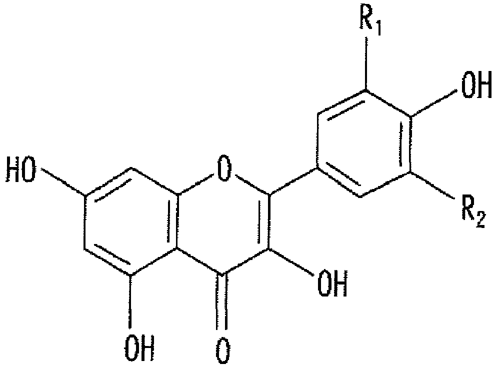
Kaempferol: R₁=H; R₂=H
Quercetin: R₁=OH; R₂=H
Myricetin: R₁=OH; R₂=OH
They are flavonoid compounds which are widely distributed in plants. These flavonols are known to have an antioxidant effect, and are reported to have a blood vessel strengthening effect and anti-inflammatory effect. However, it has not been known that flavonols can enhance in vivo expression of a redox-associated factor. Further, it is surprising that kaempferol and quercetin can enhance the expression of a redox-associated factor among these flavonols, which are quite similar in chemical structure. Taking into consideration such knowledge, it is clear that kaempferol and quercetin are quite useful as an active ingredient for enhancing the expression of a redox-associated factor.

In this context, since it is well known that flavonols may form various glycosides thereof, glycosides of kaempferol or quercetin can be also used as an active ingredient in the present agent for enhancing the expression of a redox-associated factor. Saccharides forming the glycoside according to the invention include, but are not limited to, aldoses, e.g., glucose, mannose, galactose, fucose, rhamnose, arabinose, and xylose, and ketoses, e.g., fructose.

As described above, since kaemperol, quercetin and glycosides thereof are ingredients widely distributed in plants, plant extracts comprising on of the ingredients are also effective for enhancing in vivo expression of a redox-associated factor. Among many plant extracts, the inventors now discovered that, in particular, ginkgo leaf extract and Kaempferia galanga extract have a remarkable effect for enhancing the expression of a redox-associated factor. Therefore, another embodiment of the invention provides an agent for enhancing expression of a redox-associated factor, which comprises ginkgo leaf extract, Kaempferia galanga extract, or any combination thereof as an active ingredient.

A blended amount of kaempferol, quercetin or glycosides thereof, or any combination thereof which is used as an active ingredient in the present agent for enhancing the expression of a redox-associated factor is not limited, but is usually 0.00001 to 0.5wt%, preferably 0.0001 to 0.001wt%, and most preferably 0.0001 to 0.0005wt%. Further, a blended amount of ginkgo leaf extract, Kaempferia galanga extract, or any combination thereof which is used as an active ingredient in the present agent for enhancing the expression of a redox-associated factor is, for example 0.00001 to 0.5wt%, preferably 0.0001 to 0.1wt%, and most preferably 0.0001 to 0.01wt% as dry weight.

In the present agent for enhancing the expression of a redox-associated factor, the above active ingredients can be orally or parenterally administered, and the agents can be prepared as, for example, an oral composition or a external composition. Further, the present agent for enhancing the expression of a redox-associated factor can maintain good health, and prevent various diseases and symptoms resulting from oxidative stress. Such diseases and symptoms are, but are not limited to, cancer; diabetes; autoimmune disease such as rheumatoid arthritis, familyic erythematodes, antiphospholipid antibody syndrome, dermatomyositis, familyic sclerosis cutanea, Sjogren's syndrome, Guillain-Barre syndrome, chronic atrophic gastritis, aortitis syndrome, Goodpasture's syndrome, rapidly progressive glomerulonephritis, Graves' disease, chronic discoid lupus erythematosus, or recurrent fetal loss; ischemic disease such as angina pectoris, cardiac infarction, cerebral infarction, or arteriosclerosis obliterans; allergic disease such as bronchial asthma, allergic rhinitis, hay fever, allergic enterogastritis, hives, contact dermatitis, or atopic dermatitis; skin aging; pigment deposition; wrinkle; seborrhea; sunburn; burn; acne; skin sag; obesity; high blood pressure; metabolic syndrome; and anemia are exemplified. Therefore, the agent for enhancing the expression of a redox-associated factor according to the present invention can be unlimitedly used as a raw material or additive in foods, beverages, cosmetics, medicaments, and quasi-medicaments or the like.

As an oral composition, for example, common food, as well as food with health-promoting benefits, supplement, food for specified health use, modified milk for premature infants, modified milk for infants, food for infants, food for pregnant women, food for elderly people, beverage, and medicament are included. The oral composition may be prepared as a pill, powder, a granule, a lozenge, oral solution, suspension, emulsion, syrup or the like.

Moreover, the present agent for enhancing the expression of a redox-associated factor may comprise various carriers and additives generally used for foods/beverages, medicaments or quasi-medicaments such as anti-oxidants, in addition to the active ingredient(s). For example, the agent for enhancing the expression of a redox-associated factor may further comprise various carrier substrates, extenders, diluents, bulking agents, dispersants, excipients, coupling agent solvents (e.g., water, ethanol, plant oil), solubilizing agents, buffers, dissolution promoters, gelling agents, suspending agents, wheat flour, rice flour, starch, cornstarch, polysaccharides, milk proteins, collagen, rice oil, lecithin or the like, in addition to the active ingredient of the present invention. As additives, for example, vitamins, sweeteners, organic acids, coloring agents, fragrance, moisture preventers, fiber, electrolytes, minerals, nutrients, antioxidants, preservatives, aromatic agents, moisturizers, and natural food extracts and vegetable extracts are exemplified, but not limited to them. In addition, as antioxidants, for example, natural antioxidants such as tocopherols, flavone derivatives, phyllodulcin compounds, kojic acid, gallic acid derivatives, catechin compounds, fukiic acid, gossypol, pyrazine derivatives, sesamol, guaiaol, guaiacic acid, p-coumalic acid, nor-dihydroguaiaretic acid, sterol compounds, terpene compounds, nucleic acid base compounds, carotenoid compounds and lignan compounds; and synthetic antioxidants such ascorbic palmitate, ascorbic stearate, butylhydroxyanisole (BHA), butylhydroxytoluene (BHT), mono-t-butylhydroquinone (TBHQ) and 4-hydroxymethyl-2,6-di-t-butylphenol (HMBP) are exemplified.

As an external composition, for example, ointment, cream, protector, adhesive skin patch, pack, toner, milky lotion, lotion, bath agent, hair lotion, hair tonic, hair liquid, shampoo and rinse are included. Further, an ingredient commonly used for an external agent, for example, whitening agent, moisturizer, oily ingredient, UV absorber, surfactant, thickening agent, alcohols, powder component, coloring material, aqueous ingredient, water, and various skin nutrients can be optionally blended in the present agent for enhancing the expression of a redox-associated factor. In addition, an auxiliary agent commonly used for an external preparation, for example, a metal-chelate agent such as disodium edetate, trisodium edetate, sodium citrate, sodium polyphosphates, sodium metaphosphate, gluconic acid; caffeine, tannin, verapamil, tranexamic acid and their derivatives; a medical agent such as licorice extract, glabridin, hot water extract of Chinese quince fruit, various herbal medicines, tocopherol acetate, glycyrrhizinate, and their derivatives, or their salts; a whitening agent such as vitamin C, ascorbic acid magnesium phosphate, ascorbic acid glucoside, arbutin, kojic acid; a saccharide such as glucose, fructose, mannose, sucrose, and trehalose; vitamin A such as retinoic acid, retinol, retinol acetate, and retinol palmitate can be optionally blended in the present agent for enhancing the expression of a redox-associated factor.

Representative formulations of the present agents for enhancing the expression of a redox-associated factor are shown below.

| Protector A | |
|---|---|
| Composition | Blend ratio (wt%) |
| Decamethylcyclopentasiloxane | 3 |
| Methyl phenyl polysiloxane | 3 |
| Behenyl alcohol | 1 |
| 1,3-butylene glycol | 5 |
| Polyoxyethylene glyceryl isostearate | 1.5 |
| Polyoxyethylene-glycerin monostearate | 1 |
| Ginkgo leaf extract (dried residue) as an agent for enhancing expression of redox-associated factors | 0.0001 |
| Quercetin as an agent for enhancing expression of redox-associated factors | 0.0001 |
| Trisodium edentate | 0.1 |
| 2-ethylhexyl p-methoxycinnamate | 7 |
| Xanthan gum | 0.1 |
| Carboxy vinyl polymer | 0.3 |
| Phenoxyethanol | adequate |
| Purified water | adequate |
| Fragrance | adequate |

| Protector B | |
|---|---|
| Composition | Blend ratio (wt%) |
| Petrolatum | 1 |
| Dimethylpolysiloxane | 3 |
| Methyl phenyl polysiloxane | 3 |
| Stearyl alcohol | 0.5 |
| Glycerin | 7 |
| Dipropylene glycol | 3 |
| 1,3-butylene glycol | 7 |
| Xylitol | 3 |
| Squalane | 1 |
| Isostearic acid | 0.5 |
| Stearic acid | 0.5 |
| Polyoxyethylene-glycerin monostearate | 1 |
| Glycerin monostearate | 2 |
| Potassium hydroxide | 0.05 |
| K. galanga extract (dried residue) as an agent for enhancing expression of redox-associated factors | 0.0001 |
| Kaempferol as an agent for enhancing expression of redox-associated factors | 0.0001 |
| Sodium acetylhyaluronate | 0.1 |
| Trisodium EDTA | 0.05 |
| 4-t-butyl-4'-methoxydibenzoylmethane | 2 |
| 2-ethylhexyl p-methoxycinnamate | 5 |
| Carboxy vinyl polymer | 0.1 |
| Phenoxyethanol | adequate |
| Purified water | adequate |
| Fragrance | adequate |

| Two-layer type cream | |
|---|---|
| Composition | Blend ratio (wt%) |
| Dimethylpolysiloxane | 5 |
| Decamethylcyclopentasiloxane | 25 |
| Trimethylsiloxysilicate. | 5 |
| Copolymer of polyoxyethylene/methylpolysiloxane | 2 |
| Dipropylene glycol | 5 |
| Particulate zinc oxide (60nm) coated with dextrin palmitate | 15 |
| Ginkgo leaf extract (dried residue) as an agent for enhancing expression of redox-associated factors | 0.01 |
| Kaempferol as an agent for enhancing expression of redox-associated factors | 0.0005 |
| Paraben | adequate |
| Phenoxyethanol | adequate |
| Trisodium edetate | adequate |
| 2-ethylhexyl p-methoxycinnamate | 7.5 |
| Dimethyl distearyl ammonium hectorite | 0.5 |
| Globular alkyl polyacrylate powder | 5 |
| Butyl ethyl propanediol | 0.5 |
| Purified water | adequate |
| fragrance | adequate |

| Ointment | |
|---|---|
| Composition | Blend ratio (wt%) |
| Ginkgo leaf extract (dried residue) as an agent for enhancing expression of redox-associated factors | 0.01 |
| Kaempferol as an agent for enhancing expression of redox-associated factors | 0.001 |
| Quercetin as an agent for enhancing expression of redox-associated factors | 0.0001 |
| Stearyl alcohol | 18.0 |
| Vegetable wax | 20.0 |
| Polyoxyethylene (20) monooleate ester | 0.25 |
| Glycerin monostearate ester | 0.3 |
| Petrolatum | 40.0 |
| Purified water | balance |

| Adhesive skin patch | |
|---|---|
| Composition | Blend ratio (wt%) |
| K. galanga extract (dried residue) as an agent for enhancing expression of redox-associated factors | 0.0001 |
| Kaempferol as an agent for enhancing expression of redox-associated factors | 0.0001 |
| Quercetin as an agent for enhancing expression of redox-associated factors | 0.0001 |
| Crotamiton | 3.2 |
| Panasate 875^{®} | 2.5 |
| Squalane | 1.0 |
| dl-camphor | 0.07 |
| Polyoxyethylene (60E.O.)hydrogenated castor oil | 1.2 |
| Concentrated glycerin | 5.0 |
| Gelatin | 1.2 |
| Polyvinylpyrrolidone K-90 | 0.6 |
| Methylparaben | adequate |
| d-sorbitol solution | 35.0 |
| Aluminum hydroxide | 0.2 |
| Urea | 1.3 |
| Sodium sulfite | adequate |
| Sodium edetate | adequate |
| Citric acid | adequate |
| Hivis Wako 104^{®} | 0.22 |
| Sodium polyacrylate | 0.24 |
| Sodium carboxymethylcellulose | 2.8 |
| Kaolin | 1.0 |
| Purified water | balance |

| Iontophoresis agent | |
|---|---|
| Composition | Blend ratio (wt%) |
| Ginkgo leaf extract (dried residue) as an agent for enhancing expression of redox-associated factors | 0.0001 |
| K. galanga extract (dried residue) as an agent for enhancing expression of redox-associated factors | 0.0001 |
| Kaempferol as an agent for enhancing expression of redox-associated factors | 0.0001 |
| Quercetin as an agent for enhancing expression of redox-associated factors | 0.0001 |
| Glycerin | 5.0 |
| 1,3-butylene glycol | 5.0 |
| Hyaluronic acid | 0.05 |
| Citric acid (food) | 0.35 |
| Ion-exchange water | balance |

| Candy A | |
|---|---|
| Composition | Blend ratio (wt%) |
| Ginkgo leaf extract (dried residue) as an agent for enhancing expression of redox-associated factors | 0.5 |
| Sugar | 50.000 |
| Starch syrup | balance |
| Flavor | 1.000 |

**Tablet C**

| Composition | Blend ratio (wt%) |
|---|---|
| K. galanga extract (dried residue) as an agent for enhancing expression of redox-associated factors | 0.1 |
| Quercetin as an agent for enhancing expression of redox-associated factors | 0.5 |
| Sucrose esters | 4.667 |
| Methylcellulose | 2.400 |
| Glycerin | 1.667 |
| N-acetylglucosamine | 24.7998 |
| Hyaluronic acid | 20.000 |
| Vitamin E | 12.000 |
| Vitamin B6 | 1.333 |
| Vitamin B2 | 0.667 |
| α-lipoic acid | 1.333 |
| Coenzyme Q10 | 2.667 |
| Ceramide (Amorphophallus konjac extract) | 3.333 |
| L-proline | 20.000 |
| Crystalline cellulose | balance |

| Soft capsule B | |
|---|---|
| Composition | Blend ratio (wt%) |
| Ginkgo leaf extract (dried residue) as an agent for enhancing expression of redox-associated factors | 0.1 |
| Kaempferol as an agent for enhancing expression of redox-associated factors | 0.001 |
| Quercetin as an agent for enhancing expression of redox-associated factors | 0.0001 |
| Edible soybean oil | 35.333 |
| Royal jelly | 16.4668 |
| Maca | 2.000 |
| GABA | 2.000 |
| Bees wax | 4.000 |
| Gelatin | balance |
| Glycerin | 8.000 |
| Glycerin fatty acid esters | 7.000 |

| Granule A-B | |
|---|---|
| Composition | Blend ratio (wt%) |
| Ginkgo leaf extract (dried residue) as an agent for enhancing expression of redox-associated factors | 0.01 |
| K. galanga extract (dried residue) as an agent for enhancing expression of redox-associated factors | 0.02 |
| Kaempferol as an agent for enhancing expression of redox-associated factors | 0.001 |
| Soybean isoflavones | 42.2998 |
| Hydrogenated lactose | 45.000 |
| Soybean oligosaccharides | 3.000 |
| Erythritol | 3.000 |
| Dextrin | 2.500 |
| Citric acid | 2.000 |
| Flavor | balance |

| Drink A | |
|---|---|
| Composition | Blend ratio (wt%) |
| Ginkgo leaf extract (dried residue) as an agent for enhancing expression of redox-associated factors | 0.001 |
| K. galanga extract (dried residue) as an agent for enhancing expression of redox-associated factors | 0.001 |
| Kaempferol as an agent for enhancing expression of redox-associated factors | 0.0001 |
| Quercetin as an agent for enhancing expression of redox-associated factors | 0.0001 |
| Hydrogenated maltose syrup | 65.3998 |
| Erythritol | 16.000 |
| Citric acid | 4.000 |
| Flavor | 2.600 |
| N-acetylglucosamine | 2.000 |
| Hyaluronic acid | 1.000 |
| Vitamin E | 0.600 |
| Vitamin B6 | 0.400 |
| Vitamin B2 | 0.200 |
| α-lipoic acid | 0.400 |
| Coenzyme Q10 | 2.400 |
| Ceramide (Amorphophallus konjac extract) | 0.800 |
| L-proline | 4.000 |
| Purified water | balance |

| Cookie | |
|---|---|
| Composition | Blend ratio (wt%) |
| Ginkgo leaf extract (dried residue) as an agent for enhancing expression of redox-associated factors | 0.5 |
| Kaempferol as an agent for enhancing expression of redox-associated factors | 0.5 |
| Soft wheat flour | 48.7998 |
| Butter | 17.500 |
| Granulated sugar | 20.000 |
| Egg | 12.500 |
| Flavor | balance |

Granulated sugar is gradually added to a stirred butter, followed by egg, ginkgo leaf extract, K. galanga extract, kaempferol, quercetin and flavor ingredient, and further stirred. After sufficiently blending the mixture, homogeneously-dispersed soft wheat flour is added, the mixture is gently stirred, and the blended dough is placed in a refrigerator. Subsequently, the cooled dough is shaped, and then baked at 170°C for 15 minutes to make cookies.

### EXAMPLES

### Example 1: Search for an agent enhancing the expression of thioredoxin superfamily

Normal human epidermal keratinocytes (KK-4009, KURABO) were cultured in Defined Keratinocyte-SFM medium (10744-019, GIBCO) with 5% CO₂ under high humidity at 37°C. For the cells with 100% of final density, the medium was replaced with EpiLife™ Calcium-Free Phenol Red Free medium (M-EPICF/PRF-500, KURABO) whose calcium concentration had been adjusted to 1.5 mM. Twenty-four hours later, kaempferol (K0018, Tokyo Chemical Industry Co., Ltd), vitamin E (95240, FULKA), or lipoic acid ((+/- )-α-lipoic acid (T1395, SIGMA) was so as to have a final concentration of 10 µM.

Twenty-four hours after adding the above agents, RNA was isolated by using QIAZOL Lysis Reagent (79306, QIAGEN) and purified by using RNeasy Kit (74106, QIAGEN). Subsequently, mRNAs of thioredoxin reductase, thioredoxin, glutathione reductase, glutaredoxin, and the ribosomal protein as an internal standard were quantified by the LightCycler software (Roche) using QuantiTect SYBR Green RT-PCR Kit (204243, QIAGEN). The Sequences of the used primers are shown below.
Thioredoxin reductase/Forward primer:
TGCTGGCAATAGGAAGAGATGGCTTGCAC
Thioredoxin reductase/ Reverse primer:
GCAATCTTCCTGCCTGCCTGGATTGCAACTGG
Thioredoxin/Forward primer:
TCGGTCCTTACAGCCGCTCGTCAGACTCCA
Thioredoxin/Reverse primer:
AGGCCCACACCACGTGGCTGAGAAGTCAAC
Glutathione reductase/Forward primer:
GATCCTGTCAGCCCTGGGTTCTAAGACATC
Glutathione reductase/Reverse primer:
TAACCATGCTGACTTCCAAGCCCGACAA
Glutaredoxin/Forward primer:
ATCACAGCCACCAACCACACTAACGAGA
Glutaredoxin/Reverse primer:
GTTACTGCAGAGCTCCAATCTGCTTTAGCC
A ribosomal protein/ Forward primer:
ACAGAGGAAACTCTGCATTCTCGCTTCCTG
A ribosomal protein/Reverse primer:
CACAGACAAGGCCAGGACTCGTTTGTACC
The mRNA amounts of thioredoxin reductase, thioredoxin, glutathione reductase and glutaredoxin were divided by the mRNA amount of the ribosomal protein to obtain the mRNA expression level.

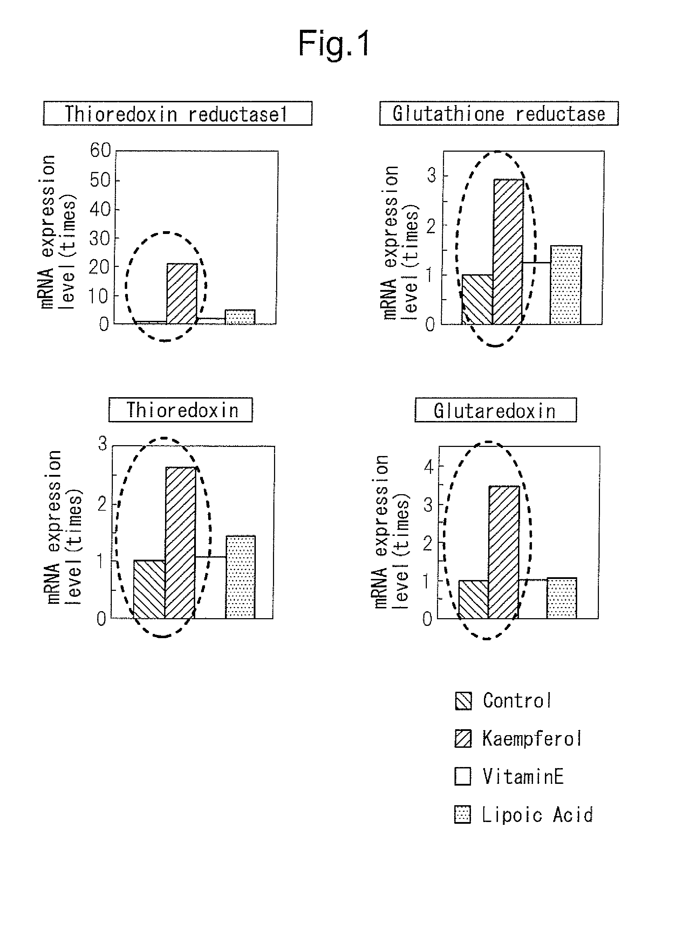

As a result, kaempferol significantly enhanced the expression of the redox-associated factors, compared with vitamin E and lipoic acid which are known to have an antioxidant effect (Fig.1).

### Example 2: Effect of kaempferol in enhancing the expression of redox-associated factors in skin-equivalent model

Skin-equivalent models were made from normal human keratinocytes (Invitrogen), normal human fibroblats (ATCC) and collagen I (Sigma), and cultured in Dulbecco's Modified Eagle's Medium (D-MEM) (Invitrogen) with 5% CO₂ under high humidity at 37°C. 2-µM or 10µM kaempferol was added into the culture medium and the models were cultured for twenty-four hours. Then RNA was isolated from epidermal layer of the skin-equivalent models by using a QIAGEN mRNA isolation kit. Subsequently, mRNAs of thioredoxin reductase and glutathione reductase, and 36B4 as an internal standard were quantified by q-PCR (quantitative PCR by Bio-Rad MyiQ single color real time PCR detection system) using the Real-time PCR kit (Bio-Rad). The primers were purchased from QIAGEN (TXNRD2: #QT00070371; GSR; #QT00038325). The amounts of thioredoxin reductase and glutathione reductase were divided by the mRNA amount of 36B4 to obtain the mRNA expression level.

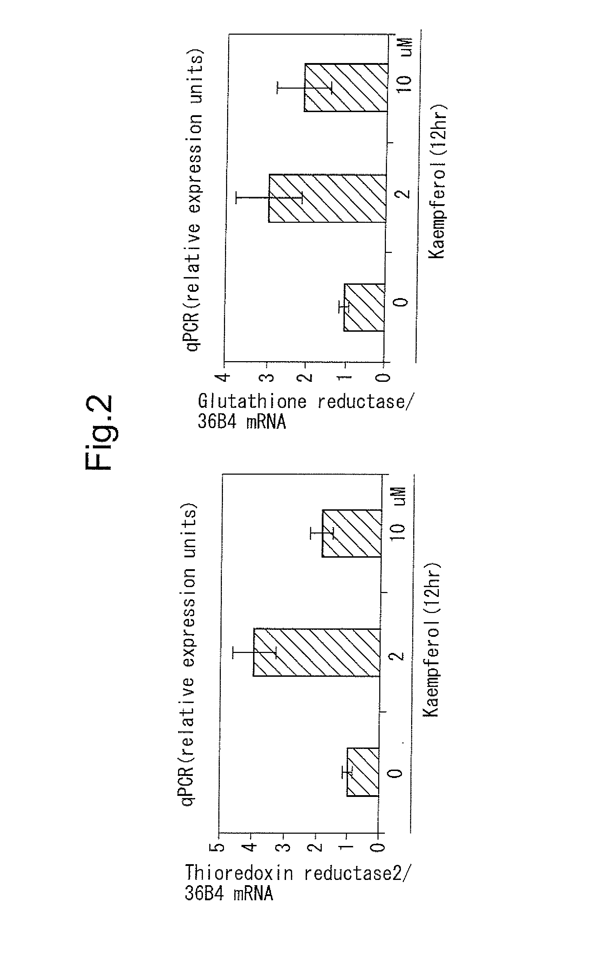

As a result, kaempferol significantly enhanced the expression of the redox-associated factors in skin-equivalent models (Fig.2).

### Example 3: Search for an agent enhancing the expression of thioredoxin superfamily - flavonoid compounds

Normal human epidermal keratinocytes (KK-4009, KURABO) were cultured in Defined Keratinocyte-SFM medium (10744-019, GIBCO) with 5% CO₂ under high humidity at 37°C. For the cells with 100% of final density, the medium was replaced with EpiLife™ Calcium-Free Phenol Red Free medium (M-EPICF/PRF-500, KURABO) whose calcium concentration had been adjusted to 1.5 mM. Twenty-four hours later, each of the following agents was added so as to have a final concentration of 10 µM.
Kaempferol (K0018, Tokyo Chemical Industry Co., Ltd)
Quercertin (173-00403, Wako Pure Chemical Industries, Ltd.)
Myricetin (70050, FLUKA)
Epigallocatechin gallate (E4143, CIAL)
Hesperidin (086-07342, Wako Pure Chemical Industries, Ltd.)
Apigenin (012-18913, Wako Pure Chemical Industries, Ltd.)
Genistein (070-04681, Wako Pure Chemical Industries, Ltd.)
Twenty-four hours after adding the above agents, the mRNA expression levels of the redox-associated factors were quantified by the same method as Example 1.

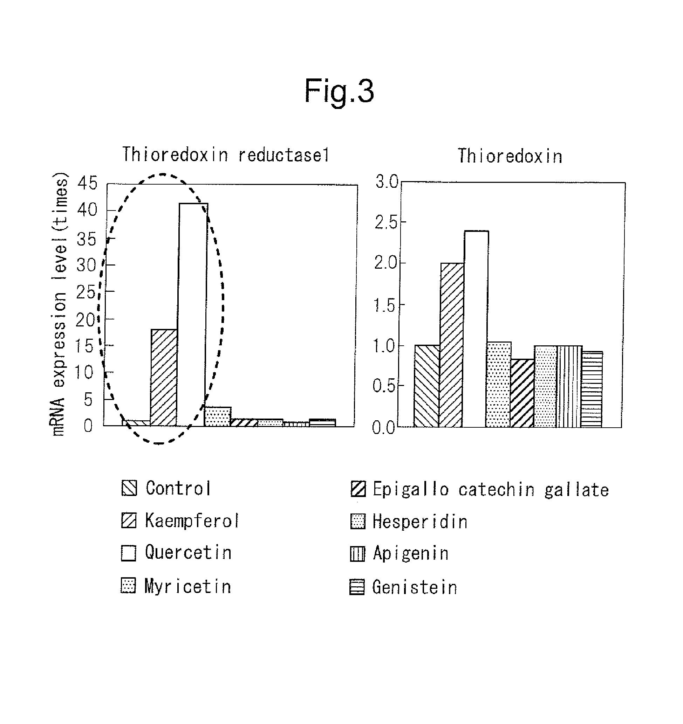

As a result, among the above flavonoid compounds, only kaempferol and quercetin significantly enhanced the expression of the thioredoxin superfamily (Fig.3).

### Example 4: Search for an agent enhancing the expression of thioredoxin superfamily - search for a plant extract

Normal human epidermal keratinocytes (KK-4009, KURABO) were cultured in Defined Keratinocyte-SFM medium (10744-019, GIBCO) with 5% CO₂ under high humidity at 37°C. For the cells with 100% of final density, the medium was replaced with EpiLife™ Calcium-Free Phenol Red Free medium (M-EPICF/PRF-500, KURABO) whose calcium concentration had been adjusted to 1.5 mM. Twenty-four hours later, each of the following agents was added so as to have a final concentration of 0.1%.
K. galanga extract: which is obtained by the extraction of Kaempferia galanga rhizomes with 50% 1,3-butylene glycol;
Ginkgo leaf extract: which is obtained by resolving 50% ethanol extract from Ginkgo biloba L. leaves in 1,3-butylene glycol;
Rubus suavissimus extract: which is obtained by resolving boiling-water extract from Rubus suavissimus Shugan Lee. (Rosaseae) in 50% 1,3-butylene glycol.
Twenty-four hours after adding the above agents, the mRNA expression levels of the redox-associated factors were quantified by the same method as Example 1.

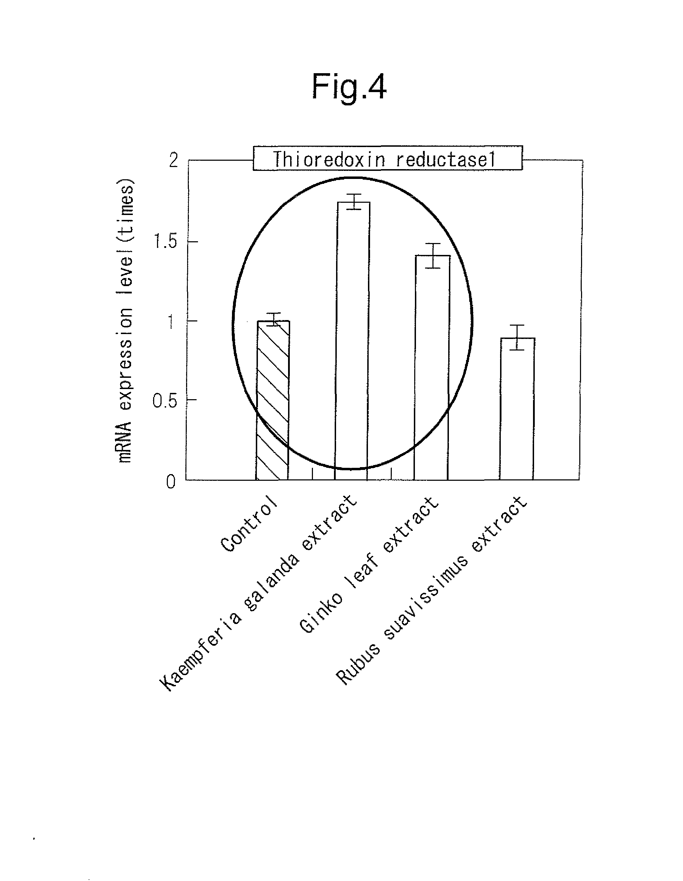

As a result, among the plant extracts containing kaempferol, quercetin and/or glycosides thereof, it was verified that ginkgo leaf extract and K. galanga extract particularly enhanced the expression of the thioredoxin superfamily (Fig.4).

### Example 5: Thioredoxin reductase activity-enhancing effect

Normal human epidermal keratinocytes (KK-4009, KURABO) were cultured in Defined Keratinocyte-SFM medium (10744-019, GIBCO) with 5% CO₂ under high humidity at 37°C. For the cells with 100% of final density, the medium was replaced with EpiLife™ Calcium-Free Phenol Red Free medium (M-EPICF/PRF-500, KURABO) whose calcium concentration had been adjusted to 1.5 mM. Twenty-four hours later, kaempferol (K0018, Tokyo Chemical Industry Co., Ltd) or quercertin (173-00403, Wako Pure Chemical Industries, Ltd.) was added so as to have a final concentration of 10 µM. Twenty-four hours after adding the agents, the cells were dissolved in lysis buffer, and the insoluble fraction was removed by centrifugation at 14,000 rpm, 4°C for 15min. The amount of total protein in the soluble fraction was quantified, and then thioredoxin reductase activity was measured by the method of Arne Holmgren et al. (METHODS IN ENZYMOLOGY, 1995, 252, 199-).

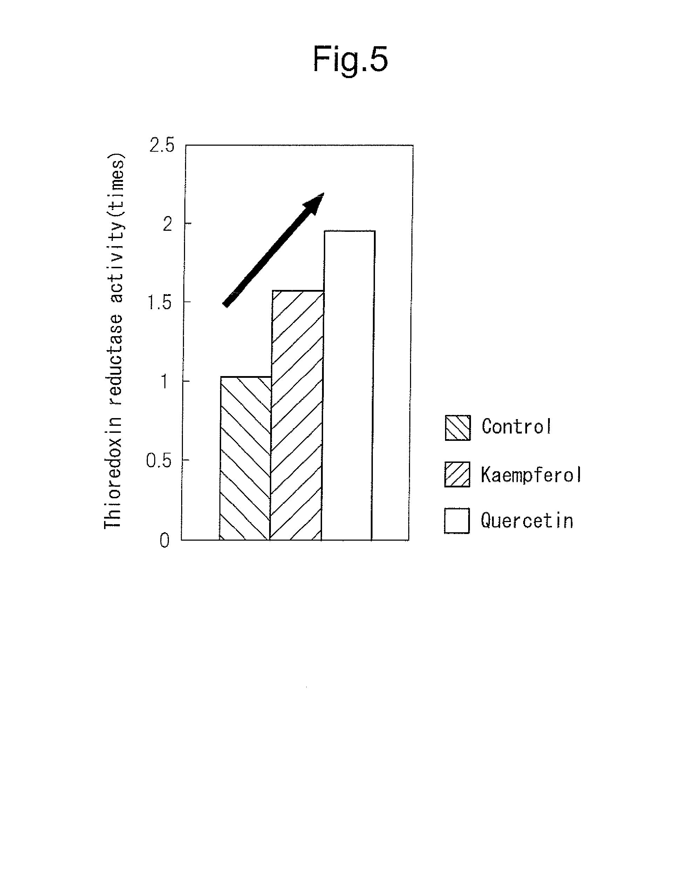

As a result, it was verified that kaempferol and quercetin enhanced thioredoxin reductase activity (Fig.5).

### Example 6: Suppression effects of kaempferol, K. galanga extract and ginkgo leaf extract on the expression of an inflammatory cytokine (IL1β)

Normal human epidermal keratinocytes (KK-4009, KURABO) were cultured in Defined Keratinocyte-SFM medium (10744-019, GIBCO) with 5% CO₂ under high humidity at 37°C. For the cells with 100% of final density, the medium was replaced with EpiLife™ Calcium-Free Phenol Red Free medium (M-EPICF/PRF-500, KURABO) in which calcium concentration had been adjusted to 1.5 mM. Twenty-four hours later, kaempferol was added to have 10 µM of final concentration and each of the following plant extracts was added to have a final concentration of 0.1%.
kaempferol (K0018, Tokyo Chemical Industry Co., Ltd);
K. galanga extract: which is obtained by the extraction of Kaempferia galanga rhizomes with 50% 1,3-butylene glycol;
Ginkgo leaf extract: which is obtained by resolving 50% ethanol extract from Ginkgo biloba L. leaves in 1,3-butylene glycol.
Twenty-four hours after adding the above agents, RNA was isolated by the same method as Example 1, and the mRNA expression level of the inflammation-associated factor (IL1β) was quantified. The used primers are shown below.
IL1β/Forward primer
GGCCATGGACAAGCTGAGGAAGATGCTG
IL1β/Reverse primer
TGCATCGTGCACATAAGCCTCGTTATCCC
The mRNA amount of IL1β was divided by the mRNA amount of the ribosomal protein to obtain the mRNA expression level.

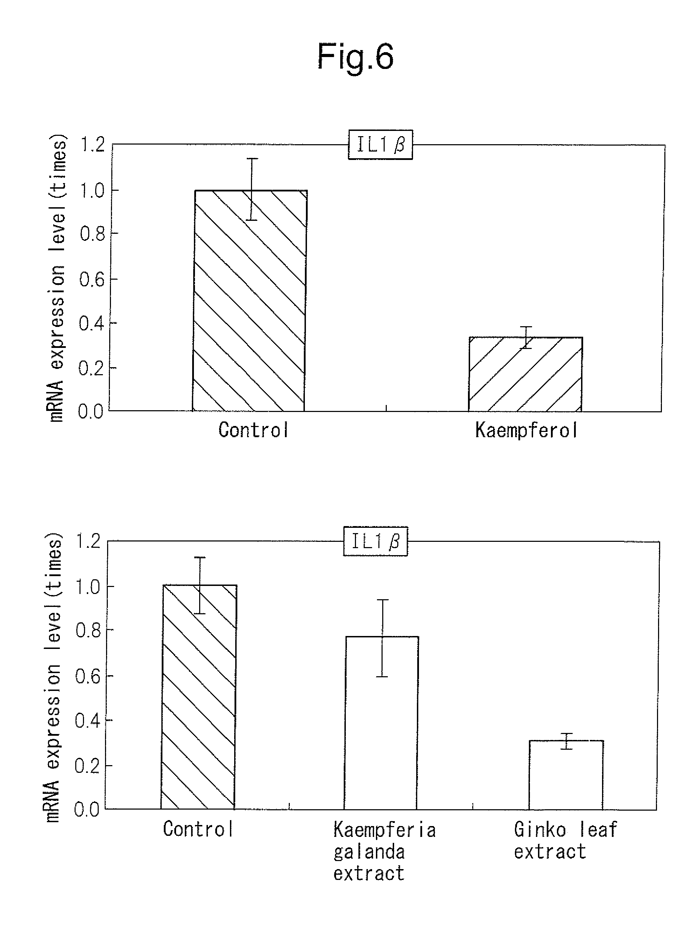

As a result, it was verified that kaempferol, K. galanga extract and ginkgo leaf extract have an anti-inflammatory effect because the production of the inflammatory cytokine IL1β was suppressed by these ingredients (Fig.6).

## Claims

1. An agent for enhancing expression of a redox-associated factor, which comprises kaempferol, quercetin, glycosides thereof or any combination thereof as an active ingredient.

2. The agent for enhancing expression of a redox-associated factor according to claim 1, wherein the redox-associated factor is one or more substances selected from the group consisting of thioredoxin, thioredoxin reductase, glutathione reductase, and glutaredoxin.

3. An agent for enhancing expression of a redox-associated factor, which comprises ginkgo leaf extract, Kaempferia galanga extract, or any combination thereof as an active ingredient.

4. The agent for enhancing expression of a redox-associated factor according to claim 3, wherein the redox-associated factor is one or more substances selected from the group consisting of thioredoxin, thioredoxin reductase, glutathione reductase, and glutaredoxin.
